# FASCICULE DE BREVET EUROPEEN

(11) **EP 0 589 993 B1**
(45) Date de publication et mention de la délivrance du brevet: **26.05.1999**
(21) Numéro de dépôt: 92912543.3
(22) Date de dépôt: 16.06.1992
(51) Int. Cl.: C12N 15/53, C07K 7/04, C12P 21/08, G01N 33/576, A61K 39/395, G01N 33/68

(54) **FRAGMENTS PEPTIDIQUES ISSUS DU CYTOCHROME HUMAIN P450 IID6, ANTICORPS ANTI-FRAGMENTS PEPTIDIQUES ET LEURS APPLICATIONS DANS LE DIAGNOSTIC DE L'HEPATITE AUTO-IMMUNE**
PEPTIDFRAGMENTE DES MENSCHLICHEN CYTOCHROME P450 IID6, ANTIKÖRPER DAGEGEN UND IHRE VERWENDUNGEN IN DIAGNOSTIK
HUMAN P450 IID6 CYTOCHROME-DERIVED PEPTIDE FRAGMENTS, ANTI PEPTIDE FRAGMENT ANTIBODIES, AND APPLICATIONS THEREOF IN THE DIAGNOSIS OF AUTOIMMUNE HEPATITIS

(30) Priorité: 17.06.1991 FR 9107363
(43) Date de publication de la demande: 06.04.1994
(73) Titulaire: INSTITUT NATIONAL DE LA SANTE ET DE LA RECHERCHE MEDICALE (INSERM), 75654 Paris Cédex 13 (FR)
(72) Inventeur: ALVAREZ, Fernando, F-92190 Meudon (FR)
(74) Mandataire: Ores, Irène
(86) Numéro de dépôt international: FR9200539
(87) Numéro de publication internationale: WO9222656

(56) Documents cités:
- PROCEEDINGS OF THE NATIONAL ACADEMY OF SCIENCES OF USA. vol. 85, Novembre 1988, WASHINGTON US pages 8256 - 8260; U.R. ZANGER ET AL: 'Antibodies against human cytochrome P-450db1 in autoimmune hepatitis type II' cité dans la demande
- BIOCHEMICAL AND BIOPHYSICAL RESEARCH COMMUNICATIONS. vol. 159, no. 2, 15 Mars 1989, DULUTH, MINNESOTA US pages 542 - 547; M. GUEGUEN ET AL: 'Anti-liver kidney microsome antibody type I recognizes human cytochrome P450 db1' cité dans la demande
- THE JOURNAL OF CLINICAL INVESTIGATION vol. 88, no. 4, Octobre 1991, NEW YORK pages 1370 - 1378; M. P. MANNA ET AL: 'LKM-1 Autoantibodies recognize a short linear sequence in P450IID6, a cytochrome P-450 Monooxygenase'

## Description

La présente invention est relative à des fragments peptidiques issus du cytochrome P450 IID6 (anciennement dénommé cytochrome P450 dbl) humain, à des anticorps anti-fragments peptidiques et à leurs applications dans le diagnostic de l'hépatite auto-immune et plus particulièrement dans le diagnostic différentiel entre l'hépatite auto-immune et d'autres hépatites chroniques d'origine virale, telles que l'hépatite C ou l'hépatite B.

L'hépatite auto-immune, en particulier chez l'enfant, est une maladie inflammatoire qui progresse vers la cirrhose et l'insuffisance hépatique, qui répond généralement à un traitement immunosuppresseur et est caractérisée par la présence de titres élevés d'auto-anticorps non spécifiques d'organe.

Deux sous-groupes ont été définis, en fonction de l'auto-anticorps présent dans le sérum : anticorps anti-muscle lisse (anti-SMA) et anticorps anti-foie-rein-microsome (anti-LKM), dénommés ci-après anticorps anti-LKM.

L'antigène reconnu par les anticorps anti-LKM est une protéine de poids moléculaire de 50 kDa, présente à une concentration relativement élevée dans le *réticulum* endoplasmique. Plusieurs études suggèrent que cet antigène correspond à une protéine de la famille du cytochrome P450 (WAXMAN B. J. et al., Gastroentérol. 1988, 95, 1326). Ceci a été confirmé par le criblage d'une banque d'ADNc de foie de rat en présence d'un anticorps anti-protéine 50KDa, purifié par affinité à partir d'un sérum LKM positif. Deux formes constitutives du cytochrome P450, appartenant à la sous-famille IID 1 et 2 (dbl et db2 de rat) ont été identifiées, comme les antigènes reconnus par l'anticorps anti-LKM (GUEGUEN M. et al., J. Exp. Med., 1988, 168, 801). D'autres études (GUEGUEN M. et al., Biochem. Biophys. Res. Commun., 1989, 159, 542 ; ZANGER U.M. et al., Proc. Natl. Acad. USA, 1988, 27, 8256 ; MANNS M.P. et al., J. Clin. Invest., 1989, 83, 1066) ont montré que le cytochrome P450 IID6 est la protéine reconnue, dans le foie humain, par l'anticorps anti-LKM.

Plusieurs méthodes de dosage des anticorps anti-LKM ont été proposées :
- une première méthode de détection de ces anticorps a été décrite dans RIZZETTO et al., 1973, Clin. Exp. Immunol., 15, 331, et a été notamment utilisée pour le diagnostic des maladies de foie associées à la production d'anticorps anti-LKM chez l'enfant (MAGGIORE et al., J. Pediatrics, 1986, 108, 3, 399-404 : *Liver-disease associated with anti-liver-kidney microsome antibody in children) ;* cette méthode consiste à détecter lesdits anticorps (sérum) par immunofluorescence indirecte sur des sections de foie et de rein de rat.

Les sérums sont considérés comme positifs pour les anticorps anti-LKM, lorsqu'ils réagissent à une dilution minimale de 1:100 avec le cytoplasme d'hépatocytes et de tubules rénaux proximaux, alors qu'aucune coloration n'est obtenue au niveau des tubules distaux.

Toutefois, cette méthode d'immunofluorescence indirecte présente l'inconvénient majeur d'être peu sensible et d'entraîner ainsi des faux négatifs qui peuvent diriger le clinicien vers un mauvais diagnostic et désorienter ainsi ce dernier par rapport aux signes cliniques observés.

De plus, les résultats obtenus sont généralement d'interprétation difficile, limitée à des laboratoires spécialisés.

D'autres tests ont par conséquent été proposés, on peut citer notamment :
- un test RIA (Clin. Exp. Immunol., 1984, 57, 600-608 : *Detection of liver-kidney microsomal auto-antibodies by radioimmunoassay and their relation to anti-mitochondrial antibodies in inflammatory liver diseases),* qui présente les inconvénients généraux des tests RIA (notamment difficulté d'obtention et de manipulation des réactifs marqués) ;
- des tests ELISA :
   . dans J. Ped. Gastoenterol. Nutr. 1988, 7, 816-822 *(Detection of anti-endoplasmic reticulum antibody-positive autoimmune hepatitis in children, using an ELISA technique)*, les Auteurs (K. PARADIS, A. DIB, JC. HOMBERG, O. BERNARD, D. ALAGILLE et F. ALVAREZ) ont décrit une méthode de détection par ELISA, utilisant, comme antigène une préparation de microsomes de foie de rat.

Cette technique s'est révélé plus sensible que l'immunofluorescence indirecte ; cependant elle a l'inconvénient d'entraîner la contamination de l'antigène utilisé par d'autres fractions, dont les mitochondries, ce qui peut entraîner des faux positifs, en particulier chez l'adulte, qui notamment dans la cirrhose biliaire primitive présente des anticorps antimitochondries.
. Poursuivant leurs travaux, les Auteurs (M. GUEGEN, AM. YAMAMOTO, O. BERNARD et F. ALVAREZ) ont remplacé les microsomes de foie, par une protéine de fusion comprenant le cytochrome P450 (Biochem. Biophys. Res. Comm., 1989, 159, 2, 542-547).

Toutefois, un tel antigène a l'inconvénient de présenter également des faux positifs, notamment en raison de la présence de la protéine associée.

Dans le but de résoudre le problème de l'obtention d'un test fiable et sensible aussi bien chez l'enfant que l'adulte (absence de faux positifs dans tous les cas), l'Inventeur, co-Auteur des articles précités, a mis au point un test de détection spécifique de l'hépatite autoimmune qui puisse notamment permettre le diagnostic différentiel avec les hépatites d'origine virale (hépatite C, notamment). En effet, il est apparu que le test de détection de l'hépatite C donne des résultats faussement positifs chez des sujets présentant, en fait, une hépatite auto-immune.

Ceci est en effet très important car les traitements des hépatites virales telles que l'hépatite B et C et de l'hépatite auto-immune sont complètement différents : dans l'hépatite B et C, on administre généralement de l'interféron recombinant, qui permet une amélioration de l'activité aminotransférase sérique, alors que dans l'hépatite auto-immune, ce sont la prednisone et l'azathioprine qui entraînent une amélioration de cette hépatite.

Il est donc important, dans la mesure où le pronostic et le traitement de ces deux types d'hépatites est radicalement différent, de pouvoir disposer d'un test et de réactifs de l'hépatite auto-immune, qui présentent notamment des propriétés diagnostiques hautement spécifiques, rapides à mettre en oeuvre et réalisables dans n'importe quel laboratoire d'analyse médicale de ville.

La présente invention s'est en conséquence donné pour but de pourvoir à des fragments peptidiques issus du cytochrome P450 humain, qui répondent mieux aux besoins de la pratique que les réactifs de l'Art antérieur, notamment en permettant la mise au point de tests diagnostiques hautement spécifiques ainsi que la préparation d'anticorps (polyclonaux et/ou monoclonaux) à visée thérapeutique et/ou diagnostique, également hautement spécifiques vis-à-vis d'au moins un épitope immuno-dominant de l'hépatite auto-immune.

La présente invention a pour objet un réactif immunologique, utilisable pour la détection, le diagnostic et le suivi de l'hépatite auto-immune, caractérisé en en ce qu'il est constitué d'un mélange de quatre peptides, dans lequel :
**(i)** le premier peptide est sélectionné dans le groupe constitué par un peptide comprenant 39 aminoacides, qui présente la séquence de formule I suivante :
   Lys-Val-Leu-Arg-Phe-Gln-Lys-Ala-Phe-Leu-Thr-Gln-Leu-Asp-Glu-Leu-Leu-Thr-Glu-His-Arg-Met-Thr-Trp-Asp-Pro-Ala-Gln-Pro-Pro-Arg-Asp-Leu-Thr-Glu-Ala-Phe-Leu-Ala (I), laquelle séquence correspond aux aminoacides 239-278 du cytochrome P450 IID6 humain, un peptide comprenant 18 aminoacides, qui présente la séquence de formule II suivante :
   Leu-Leu-Thr-Glu-His-Arg-Met-Thr-Trp-Asp-Pro-Ala-Gln-Pro-Pro-Arg-Asp-Leu (II), laquelle séquence correspond aux aminoacides 254-271 du cytochrome P450 IID6 humain et un peptide comprenant 13 aminoacides, qui présente la séquence de formule III suivante :
   Glu-His-Arg-Met-Thr-Trp-Asp-Pro-Ala-Gln-Pro-Pro-Arg (III), laquelle séquence correspond aux aminoacides 257-269 du cytochrome P450 IID6, et un peptide comprenant 3 aminoacide, qui présente la séquence de formule (IV) suivante : Thr-Trp-Asp, laquelle séquence correspond aux aminoacides 261, 262 et 263 du cytochrome P450 IID6 humain ;
**(ii)** le deuxième peptide comprend 31 aminoacides et présente la séquence de formule Va suivante : Met-Ile-Leu-His-Pro-Asp-Val-Gln-Arg-Arg-Val-Gln-Gln-Glu-Ile-Asp-Asp-Val-Ile-Gly-Gln-Val-Arg-Arg-Pro-Glu-Met-Gly-Asp-Gln-Ala (Va), laquelle séquence correspond aux aminoacides 321-351 du cytochrome P450 IID6 ;
**(iii)** le troisième peptide comprend 17 aminoacides et présente la séquence de formule IX suivante : Gly-Met-Thr-His-Met-Thr-Ser-Arg-Asp-Ile-Gly-Val-Gln-Gly-Phe-Arg-Ile (IX), laquelle séquence correspond aux aminoacides 373-389 du cytochrome P450 IID6 humain et
**(iv)** le quatrième peptide comprend 20 aminoacides et présente la séquence de formule X suivante :
   Glu-Lys-Pro-Tyr-Pro-Glu-His-Phe-Leu-Asp-Ala-Gln-Gly-His-Phe-Val-Lys-Pro-Glu-Ala (X), laquelle séquence correspond aux aminoacides 410-429 du cytochrome P450 IID6 humain, lesquels peptides incluent également ceux ne différant que par la substitution, la délétion, l'addition d'un petit nombre d'aminoacides, à condition que les séquences ainsi modifiées aient une spécificité de liaison avec les auto-anticorps anti-LKM équivalente à celles des peptides sus-mentionnés.

Au sens de la présente invention, "fragment peptidique", englobe non seulement les séquences comprenant un fragment du cytochrome P450 IID6 humain, mais également ceux n'en différant que par la substitution, la délétion, l'addition d'un petit nombre d'acides aminés, à condition que les séquences ainsi modifiées aient une spécificité de liaison avec les auto-anticorps anti-LKM équivalente à celle des fragment sus-mentionnés.

Ces peptides peuvent en particulier être préparés par synthèse, notamment par la méthode de Merrifield.

La présente invention a également pour objet un procédé de détection, de diagnostic ou de suivi de l'hépatite auto-immune, caractérisé en ce qu'il consiste à détecter les auto-anticorps éventuellement présents dans un fluide biologique tel que le sang, en mettant en présence ledit fluide biologique avec un réactif immunologique conforme à l'invention, auquel se lient les auto-anticorps anti-LKM, si de tels anticorps sont présents dans l'échantillon biologique à contrôler, la lecture du résultat étant révélée par un moyen approprié, notamment RIA, EIA ou cytométrie de flux.

Les réactifs et le procédé de détection conformes à l'invention ont l'avantage de permettre un diagnostic différentiel entre l'hépatite auto-immune et les autres hépatites d'origine virale.

La présente invention a en outre pour objet un kit ou trousse de diagnostic pour la détection, le diagnostic ou le suivi de l'hépatite auto-immune, caractérisé en ce qu'il comprend :
- un support solide approprié convenablement revêtu d'un réactif conforme à l'invention;
- au moins un flacon contenant des conjugués choisis dans le groupe qui comprend les conjugués enzyme appropriée-anticorps anti-Ig humaines appropriés ;
- des quantités ou doses appropriées d'une substance de révélation appropriée.

Dans le cas d'une méthode directe, par exemple, les anticorps anti-LKM du patient se fixent aux peptides conformes à l'invention, préalablement fixés au support solide et des conjugués enzyme appropriée-anticorps anti-Ig humains appropriés ou des conjugués enzyme appropriée-anticorps anti-fragment Fc des Ig humaines sont introduits, se lient avec les Ig du patient et sont ensuite révélés de manière appropriée.

Dans le cas d'une méthode indirecte, par exemple, des conjugués enzyme appropriée-anticorps anti-peptides conformes à l'invention entrent en compétition avec les anticorps du patient pour se lier au support solide revêtu de peptides ou d'anticorps conformes à l'invention.

Outre les dispositions qui précèdent l'invention comprend encore d'autres dispositions, qui ressortiront de la description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention, dont ils ne constituent en aucune manière une limitation.

### EXAMPLE 1 : Sélection et préparation des fragments peptidiques conformes à l'invention.

### a) Construction des plasmides d'expression pEX627-LKMHC5 et pEX627-LKMC1.

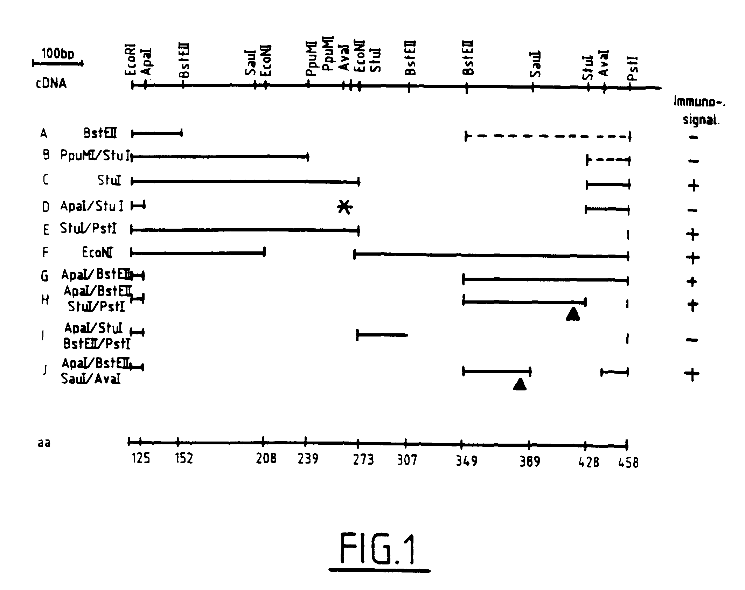

LKMHC5 est un clone d'ADNc dans le phage λGT-11, codant pour le cytochrome humain P450 IID6 (GUEGUEN M. et al. 1989, référence citée). Le fragment EcoRI-PstI d'ADNc (1007 bp) est sous-cloné dans le plasmide EX-627, qui permet l'expression d'une protéine de fusion (β-galactosidase/P450 IID6) dans la bactérie *E. coli* pop 2136. La carte de restriction du cytochrome P450 IID6 est obtenue à partir de la GenBank et utilisée pour créer différentes constructions. Les endonucléases utilisées sont : EcoRI, ApaI, BstEII, SauI, AvaI, StuI, PstI, EcoNI et PpuMI; les sites de restriction spécifiques sont représentés à la figure 1. Les protocoles utilisés pour la restriction et la ligation sont ceux décrits dans SAMBROOK et al. (Molecular cloning, Laboratory Manual, 1989, Cold Spring Harbor Laboratory). Les différentes constructions obtenues, désignées par des lettres, correspondent à différents fragments du cytochrome P450 comme suit :
- Construction A : séquence en aminoacides 125-152 ;
- Construction B : séquence 125-239 ;
- Construction C : séquence 125-273/428-458 ;
- Construction D : séquence 428-458 ;
- Construction E : séquence 125-273 (*) ;
- Construction F : séquence 125-208/273-458 ;
- Construction G : séquence 349-458 ;
- Construction H : séquence 349-428 (▲) ;
- Construction I : séquence 273-307 et
- Construction J : séquence 349-389 (▲) (voir figure 1).
Chacune de ces constructions est clonée et analysée avant l'expression, pour confirmer que la dimension prévue de l'ADN est bien obtenue.

Dans la figure 1, le signe * indique un site antigénique reconnu par les sérums de tous les malades testés et le signe ▲ indique d'autres sites antigéniques reconnus par un certain nombre de malades.
- Le clone LKMC1 est un clone d'ADNc dans le phage λGT-11, codant pour le cytochrome P450 db2 de rat. Le fragment EcoRI-BamHI de LKMC1 (800 bp) est cloné dans le plasmide pEX-627 et transfecté dans *E.coli* pop 2136. Ceci permet de tester le peptide correspondant à la région 5' du gène (codant pour l'aminoacide 1 à l'aminoacide 125).
- mise en évidence des épitopes :
   La protéine P450 IID6 complète comprend 495 aminoacides. Le clone LKMHC5 EcoRI/PSTI, tel que défini ci-dessus, qui code pour les aminoacides 125 à 458, permet l'analyse de la partie C terminale de la molécule, tandis que la région N-terminale est analysée en utilisant un sous-clone EcoRI/BamHILKMC1, tel que défini ci-dessus, et qui code pour la séquence en aminoacides 1-266 du cytochrome P450 IID6 humain.

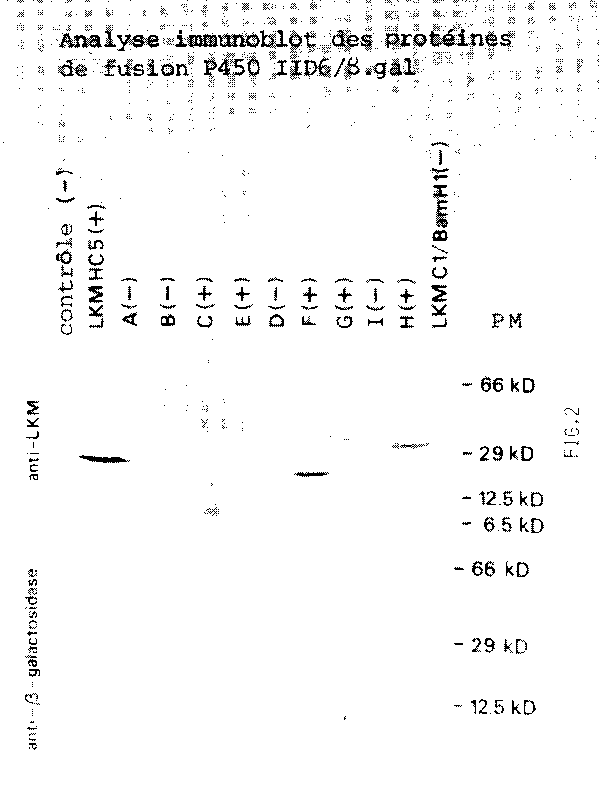

L'analyse de ces clones montre qu'il existe une homologie de 70,4 % entre la partie NH₂-terminale de 125 aminoacide de la protéine de rat P450-db2 (LKMC1) et la protéine humaine P450 IID6. Les constructions d'ADN telles que définies ci-dessus, dans le plasmide EX-627, ont permis de tester différentes régions de la protéine P450 IID6 humaine, situées entre les aminoacides 125 et 458. Sur les immunoblots, figure 2, tous les sérums positifs LKM, reconnaissent les protéines de fusion obtenues à partir des constructions C, E et F. Six sur les onze sérums testés reconnaissent les constructions G, H et J. Aucun des sérums des patients testés ne reconnait les constructions A, B, D, I et LKMC1/BamHI. Toutes les protéines de fusion sont reconnues par les anticorps anti-β-galactosidase. Ces résultats illustrent la présence d'au moins deux sites antigéniques sur la protéine humaine P450 IID6 reconnue par les anticorps anti-LKM. Les résultats combinés des constructions A(-), B(-), C(+), D(-) et E(+) montrent qu'une région peptidique de 34 aminoacides (aminoacides 239 à 273 de la protéine P450 IID6 humaine) contient au moins l'un des épitopes reconnus par tous les sérums LKM positifs testés. Puisque les constructions G, H et J sont reconnues par six des onze sérums positifs LKM testés, un autre site antigénique est probablement localisé entre les aminoacides 349 et 389. La région située entre les constructions H et I n'a pas été testée de manière spécifique mais était présente dans la construction F. Un troisième épitope est situé entre les aminoacides 307 et 349. La présence d'épitopes multiples sur un auto-antigène, est en faveur de l'hypothèse qui considère que la réponse auto-immune est polyclonale. Des résultats similaires ont été obtenus avec d'autres systèmes auto-antigènes/auto-anticorps et ceux-ci ont été interprétés comme l'absence d'une mutation faite au hasard comme le mécanisme de la production d'auto-anticorps et permettent de soutenir le fait que la réponse auto-immune soit induite par l'antigène.

### b) Expression et analyse en immunoblot des protéines de fusion en provenance des différentes constructions de pEX-627-LKMHC5 et pEX-627-LKMC1/BamHI.

L'expression des protéines de fusion à partir des construction d'ADNc est décrite par STANLEY (Nucleic Acids Research, 1983, 11, 4077). Des échantillons de 100 µl de culture, pour chaque protéine de fusion, sont soumis à une électrophorèse sur du gel de polyacrylamide-SDS 8-20%, transférés sur nitrocellulose et analysés par la technique immunoblot.

Les premiers anticorps utilisés pour la méthode immunoblot sont :
1) des sérums de patients répondant positivement aux anticorps anti-LKM ;
2) des sérums de patients répondant positivement aux anticorps anti-SMA ;
3) des anticorps anti-β-galactosidase de souris ; et
4) un sérum humain normal.

En fonction de l'origine du premier anticorps, le second anticorps est soit un conjugué peroxydase-IgG anti-humaine de chèvre, soit un conjugué peroxydase-IgG anti-souris de chèvres (Biosys), à une dilution de 1/1000.

Les microsomes de foie de rat et de foie humain et la β-galactosidase, exprimée par le plasmide pEX-627 ne comprenant pas l'insert d'ADN P450, sont inclus sur les mêmes gels en tant que contrôles positifs et contrôle négatif, respectivement.

### c) la synthèse des peptides.

la synthèse du peptide en phase solide est réalisée en utilisant un appareil Applied Biosystems. Les peptides sont synthétisés sur une résine p-benzyloxy-benzylalcool, en utilisant du 9-fluorénylméthoxycarbonyl (FMOC). Les dérivés d'aminoacides protégés sont obtenus chez Novabiochem et les autres réactifs chez Applied Biosystems. Les peptides sont scindés à partir de la résine avec un mélange acide-fluoroacétique:phénol:éthanedithiol:thioanasole:eau (80:3:1:2:2) et purifiés par chromatographie en phase inverse sur une colonne Nucléosil C8 300 A. Trois peptides sont ainsi synthétisés (séquences 241-260, 254-271 et 264-281) et ont permis d'analyser l'une des régions (241-280) de la protéine de fusion, qui est reconnue par les sérums positifs LKM (voir Tableau I ci-dessous).

Le peptide 241-260 est synthétisé avec un résidu cystéine additionnel à l'extrémité NH₂-terminale, afin d'éviter la modification de la lysine 245 (Tableau I), pendant le couplage à la sérumalbumine bovine.

**TABLEAU I**

| Peptides du cytochrome P450 IID6 | Test ELISA | Immunoblot |
|---|---|---|
| Fragment 241-260 | - | - |
| Peptide de formule II (254-271) | + | + |
| Peptide de formule III (257-269) | + | + |
| Peptide de formule IX (373-389) | + | + |
| Peptide de formule X (410-429) | + | + |
| Peptide de formule Va (321-351) | + | + |
| Fragment 264-281 | - | - |
| Fragment 347-367 | - | - |
| Fragment 359-377 | - | - |
| Fragment 383-394 | - | - |
| Fragment 382-417 | - | - |

Le site antigénique majeur localisé entre les aminoacides 239 et 273 a été analysé également en utilisant trois peptides synthétiques qui couvrent la région comprise entre les aminoacides 241 et 281 comme visible sur le Tableau I ci-dessus. L'un des peptides couvrant la région 254-271 est reconnu par tous les sérums positifs LKM lorsqu'il est testé en ELISA et en immunoblot, tandis que les trois autres peptides ne le sont pas (Va, reconnu par 8 patients/15 ; IX, reconnu par 1 patient/15 ; X, reconnu par 2 patients/15). Dans la mesure où les peptides II, III et Va synthétisés se recoupent, le tripeptide Thr-Trp-Asp représente une partie essentielle de l'épitope.

### d) couplage des peptides issus du cytochrome P450 IID6 avec la sérumalbumine bovine (BSA).

Les différents peptides et notamment les peptides 254-271 (peptide de formule II), 264-281 et 410-429 (peptide de formule X), sont couplés à la BSA par l'intermédiaire de leur groupe NH₂-terminal libre, en utilisant du glutaraldéhyde (voir notamment E. HARLOW et D. LANE, 1988, Antibodies, A laboratory manual, Cold Spring Harbor Laboratory).

Un certain nombre de peptides (241-260, 383-399, 392-417 et peptide de formule X) portent un résidu cystéine N-terminal supplémentaire qui permet le couplage à la BSA par l'intermédiaire du groupe sulfhydryle de la cystéine, en utilisant un ester de m-maléimidobenzoyl-N-hydroxysuccinimide.

### EXEMPLE 2 : Test ELISA.

Les peptides sont redissous dans un tampon PBS, pH 7,4 et dilués pour obtenir des concentrations de 0,1, 0,2, 0,5 et 1 µg/ml ; 100 µl par puits de chaque solution de peptide est incubé une nuit dans des plaques de microtitration, contenant 96 puits (Immulon 2, Dynatech, Poly-Labo, Paris - FRANCE). L'analyse ELISA est alors réalisée comme décrit dans GUEGUEN M. et al. (Biochem. Biophys. Res. Commun., 1989, 159, 542), en utilisant les différents sérums humains décrits ci-dessus. Les premiers anticorps sont testés aux dilutions allant de 1/100 à 1/12 800. L'anticorps IgG anti-humain de chèvre conjugué avec la phosphatase alcaline est utilisé comme second anticorps, à une dilution de 1/1000. Le test est développé en ajoutant 100 µl de p-nitrophénylphosphate à une concentration de 1 mg/ml, dans un tampon NaCO₃ 0,05 M, pH 9,8 et MgCl₂ 0,001 M. Les résultats lus après 30 minutes d'incubation à température ambiante sont considérés positifs lorsqu'ils sont supérieurs d'un facteur 2 par rapport au sérum contrôle.

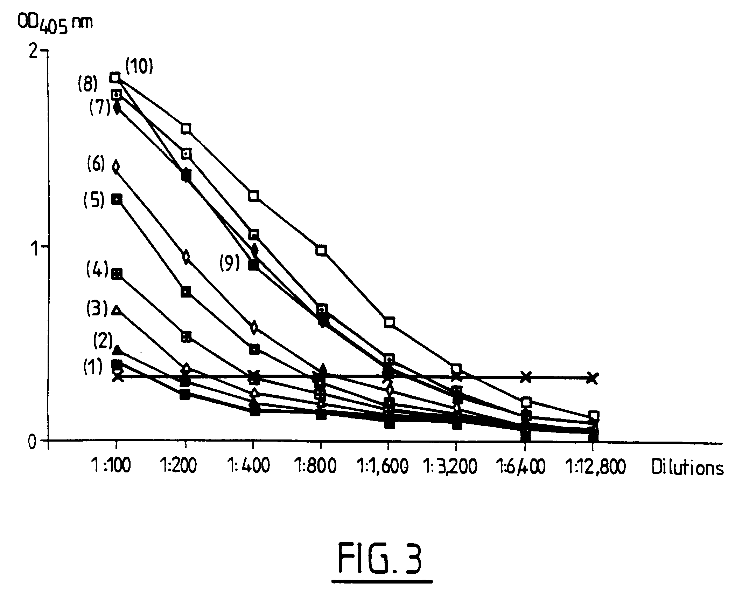

La figure 3 qui comprend en abscisse les dilutions et en ordonnée les densités optiques, illustre un test ELISA réalisé sur des sérums de 10 malades (courbes 1 à 10), avec comme antigène un peptide comprenant au moins un fragment de la séquence 239-273 (peptides de formules I, II, III ou IV) ; la courbe -x- correspond à la limite supérieure de la normale.

## Revendications

1. Réactif immunologique, utilisable pour la détection, le diagnostic et le suivi de l'hépatite auto-immune, caractérisé en ce qu'il est constitué d'un mélange de quatre peptides, dans lequel :
(i) le premier peptide est sélectionné dans le groupe constitué par un peptide comprenant 39 aminoacides, qui présente la séquence de formule I suivante :
Lys-Val-Leu-Arg-Phe-Gln-Lys-Ala-Phe-Leu-Thr-Gln-Leu-Asp-Glu-Leu-Leu-Thr-Glu-His-Arg-Met-Thr-Trp-Asp-Pro-Ala-Gln-Pro-Pro-Arg-Asp-Leu-Thr-Glu-Ala-Phe-Leu-Ala (I), laquelle séquence correspond aux aminoacides 239-278 du cytochrome P450 IID6 humain, un peptide comprenant 18 aminoacides, qui présente la séquence de formule II suivante :
Leu-Leu-Thr-Glu-His-Arg-Met-Thr-Trp-Asp-Pro-Ala-Gln-Pro-Pro-Arg-Asp-Leu (II), laquelle séquence correspond aux aminoacides 254-271 du cytochrome P450 IID6 humain, un peptide comprenant 13 aminoacides, qui présente la séquence de formule III suivante :
Glu-His-Arg-Met-Thr-Trp-Asp-Pro-Ala-Gln-Pro-Pro-Arg (III), laquelle séquence correspond aux aminoacides 257-269 du cytochrome P450 IID6, et un peptide comprenant 3 aminoacide, qui présente la séquence de formule (IV) suivante : Thr-Trp-Asp, laquelle séquence correspond aux aminoacides 261, 262 et 263 du cytochrome P450 IID6 humain ;
(il) le deuxième peptide comprend 31 aminoacides et présente la séquence de formule Va suivante : Met-Ile-Leu-His-Pro-Asp-Val-Gln-Arg-Arg-Val-Gln-Gln-Glu-Ile-Asp-Asp-Val-Ile-Gly-Gln-Val-Arg-Arg-Pro-Glu-Met-Gly-Asp-Gln-Ala (Va), laquelle séquence correspond aux aminoacides 321-351 du cytochrome P450 IID6 ;
(iii) le troisième peptide comprend 17 aminoacides et présente la séquence de formule IX suivante :
Gly-Met-Thr-His-Met-Thr-Ser-Arg-Asp-Ile-Gly-Val-Gln-Gly-Phe-Arg-Ile (IX), laquelle séquence correspond aux aminoacides 373-389 du cytochrome P450 IID6 humain et
(iv) le quatrième peptide comprend 20 aminoacides et présente la séquence de formule X suivante : Glu-Lys-Pro-Tyr-Pro-Glu-His-Phe-Leu-Asp-Ala-Gln-Gly-His-Phe-Val-Lys-Pro-Glu-Ala (X), laquelle séquence correspond aux aminoacides 410-429 du cytochrome P450 IID6 humain, lesquels peptides incluent également ceux ne différant que par la substitution, la délétion, l'addition d'un petit nombre d'aminoacides, à condition que les séquences ainsi modifiées aient une spécificité de liaison avec les auto-anticorps anti-LKM équivalente à celles des peptides sus-mentionnés.

2. Procédé de détection, de diagnostic ou de suivi de l'hépatite auto-immune, caractérisé en ce qu'il consiste à détecter les auto-anticorps éventuellement présents dans un fluide biologique tel que le sang, en mettant en présence ledit fluide biologique avec un réactif immunologique selon la revendication 1, auquel se lient les auto-anticorps anti-LKM, si de tels anticorps sont présents dans l'échantillon biologique à contrôler, la lecture du résultat étant révélée par un moyen approprié, notamment RIA, EIA ou cytométrie de flux.

3. Kit ou trousse de diagnostic pour la détection, le diagnostic ou le suivi de l'hépatite auto-immune, caractérisé en ce qu'il comprend :
- un support solide approprié convenablement revêtu d'un réactif selon la revendication 1 ;
- au moins un flacon contenant des conjugués choisis dans le groupe qui comprend les conjugués enzyme appropriée-anticorps anti-Ig humaines appropriés ;
- des quantités ou doses appropriées d'une substance de révélation appropriée.

## Claims

1. Immunological reagent usable in detecting, diagnosing and monitonng autoimmune hepatitis, characterised in that it comprises a mixture of four peptides of which:
(i) the first peptide is selected from the group comprising a peptide which comprises 39 amino acids and exhibits the following formula sequence I: Lys-Val-Leu-Arg-Phe-Gln-Lys-Ala-Phe-Leu-Thr-Gln-Leu-Asp-Glu-Leu-Leu-Thr-Glu-His-Arg-Met-Thr-Trp-Asp-Pro-Ala-Gln-Pro-Pro-Arg-Asp-Leu-Thr-Glu-Ala-Phe-Leu-Ala (I), which corresponds to amino acids 239-278 of human cytochrome P450 IID6, a peptide which comprises 18 amino acids and exhibits the following formula sequence II:
Leu-Leu-Thr-Glu-His-Arg-Met-Thr-Trp-Asp-Pro-Ala-Gln-Pro-Pro-Arg-Asp-Leu (II), which corresponds to amino acids 254-271 of human cytochrome P450 IID6, a peptide which comprises 13 amino acids and exhibits the following formula sequence III:
Glu-His-Arg-Met-Thr-Trp-Asp-Pro-Ala-Gln-Pro-Pro-Arg (III), which corresponds to amino acids 257-269 of cytochrome P450 IID6, and a peptide which comprises 3 amino acids and exhibits the following formula sequence (IV):
Thr-Trp-Asp which corresponds to amino acids 261, 262 and 263 of human cytochrome P450 IID6;
(ii) the second peptide comprises 31 amino acids and exhibits the following formula sequence Va:
Met-Ile-Leu-His-Pro-Asp-Val-Gln-Arg-Arg-Val-Gln-Gln-Glu-Ile-Asp-Asp-Val-Ile-Gly-Gln-Val-Arg-Arg-Pro-Glu-Met-Gly-Asp-Gln-Ala (Va), which corresponds to amino acids 321-351 of cytochrome P450 IID6;
(iii) the third peptide comprises 17 amino acids and exhibits the following formula sequence IX:
Gly-Met-Thr-His-Met-Thr-Ser-Arg-Asp-Ile-Gly-Val-Gln-Gly-Phe-Arg-Ile (IX), which corresponds to amino acids 373-389 of human cytochrome P450 IID6, and
(iv) the fourth peptide comprises 20 amino acids and exhibits the following formula sequence X:
Glu-Lys-Pro-Tyr-Pro-Glu-His-Phe-Leu-Asp-Ala-Gln-Gly-His-Phe-Val-Lys-Pro-Glu-Ala (X), which corresponds to amino acids 410-429 of human cytochrome P450 IID6, which peptides also include those which differ only by the substitution, deletion, addition of a small number of amino acids, on condition that the sequences thus modified have a binding specificity with anti-LKM auto-antibodies which is equivalent to those of the aforementioned peptides.

2. Process for detecting, diagnosing or monitoring autoimmune hepatitis, characterised in that it consists of detecting any auto-antibodies present in a biological fluid such as blood, by bringing together the said biological fluid with an immunological reagent according to Claim 1, to which anti-LKM auto-antibodies bind if such antibodies are present in the biological sample for testing, and reading the result thus disclosed by appropriate means, in particular RIA, EIA or flow cytometry.

3. Diagnostic kit or set for detecting, diagnosing or monitoring autoimmune hepatitis, characterised in that it comprises:
- a suitable solid substrate conveniently coated with a reagent according to Claim 1;
- at least one bottle containing conjugates selected from the group comprising the conjugates of suitable enzyme with suitable antibodies to human Ig;
- suitable quantities or doses of a suitable disclosing substance.

## Patentansprüche

1. Immunologisches Reagens, das zum Nachweis, zur Diagnose und zur Verlaufskontrolle der Autoimmunhepatitis verwendbar ist, dadurch **gekennzeichnet**, daß es aus einem Gemisch von vier Peptiden besteht, worin:
(i) das erste Peptid aus der Gruppe, bestehend aus einem Peptid, das 39 Aminosäuren umfaßt, das die Sequenz der folgenden Formel I besitzt:
Lys-Val-Leu-Arg-Phe-Gln-Lys-Ala-Phe-Leu-Thr-Gln-Leu-Asp-Glu-Leu-Leu-Thr-Glu-His-Arg-Met-Thr-Trp-Asp-Pro-Ala-Gln-Pro-Pro-Arg-Asp-Leu-Thr-Glu-Ala-Phe-Leu-Ala (I),wobei die Sequenz den Aminosäuren 239-278 von menschlichem Cytochrom P450 IID6 entspricht, einem Peptid, umfassend 18 Aminosäuren, das die Sequenz der folgenden Formel II besitzt:
Leu-Leu-Thr-Glu-His-Arg-Met-Thr-Trp-Asp-Pro-Ala-Gln-Pro-Pro-Arg-Asp-Leu (II), wobei die Sequenz den Aminosäuren 254-271 von menschlichem Cytochrom P450 IID6 entspricht, einem Peptid, umfassend 13 Aminosäuren, das die Sequenz der folgenden Formel III besitzt:
Glu-His-Arg-Met-Thr-Trp-Asp-Pro-Ala-Gln-Pro-Pro-Arg (III), wobei die Sequenz den Aminosäuren 257-269 von Cytochrom P450 IID6 entspricht, und einem Peptid, umfassend 3 Aminosäuren, das die Sequenz der folgenden Formel (IV) besitzt:
Thr-Trp-Asp, wobei die Sequenz den Aminosäuren 261, 262 und 263 von menschlichem Cytochrom P450 IID6 entspricht, ausgewählt ist;
(ii) das zweite Peptid 31 Aminosäuren umfaßt und die Sequenz der folgenden Formel Va besitzt:
Met-Ile-Leu-His-Pro-Asp-Val-Gln-Arg-Arg-Val-Gln-Gln-Glu-Ile-Asp-Asp-Val-Ile-Gly-Gln-Val-Arg-Arg-Pro-Glu-Met-Gly-Asp-Gln-Ala (Va),wobei die Sequenz den Aminosäuren 321-351 von Cytochrom P450 IID6 entspricht;
(iii) das dritte Peptid 17 Aminosäuren umfaßt und die Sequenz der folgenden Formel IX besitzt:
Gly-Met-Thr-His-Met-Thr-Ser-Arg-Asp-Ile-Gly-Val-Gln-Gly-Phe-Arg-Ile (IX), wobei die Sequenz den Aminosäuren 373-389 von menschlichem Cytochrom P450 IID6 entspricht und
(iv) das vierte Peptid 20 Aminosäuren umfaßt und die Sequenz der folgenden Formel X besitzt:
Glu-Lys-Pro-Tyr-Pro-Glu-His-Phe-Leu-Asp-Ala-Gln-Gly-His-Phe-Val-Lys-Pro-Glu-Ala (X),wobei die Sequenz den Aminosäuren 410-429 von menschlichem Cytochrom P450 IID6 entspricht, wobei die Peptide auch diejenigen umfassen, die sich nur durch Substitution, Deletion, Addition einer kleinen Anzahl von Aminosäuren unterscheiden, mit der Maßgabe, daß die so modifizierten Sequenzen eine Bindungsspezifität mit den Anti-LKM-Autoantikörpern besitzen, die derjenigen der vorstehend genannten Peptide äquivalent ist.

2. Verfahren zum Nachweis, zur Diagnose oder zur Verlaufskontrolle der Autoimmunhepatitis, dadurch **gekennzeichnet**, daß man die in einer biologischen Flüssigkeit, wie Blut, gegebenenfalls vorhandenen Autoantikörper nachweist, indem man die biologische Flüssigkeit mit einem immunologischen Reagens nach Anspruch 1 in Kontakt bringt, an das sich die Anti-LKM-Autoantikörper, sofern derartige Antikörper in der zu kontrollierenden biologischen Probe vorhanden sind, binden, das Ergebnis, das mit einem geeigneten Mittel, insbesondere RIA, EIA oder Durchfluß-Cytometrie, nachgewiesen wurde, abliest.

3. Kit oder diagnostische Reagenszusammenstellung zum Nachweis, zur Diagnose oder zur Verlaufskontrolle der Autoimmunhepatitis, dadurch **gekennzeichnet**, daß es/sie umfaßt:
- einen geeigneten festen Träger, der in zweckdienlicher Weise mit einem Reagens nach Anspruch 1 beschichtet ist;
- mindestens ein Gefäß, das Konjugate, ausgewählt aus der Gruppe, umfassend Konjugate aus einem geeigneten Enzym, mit geeigneten menschlichen Anti-Ig-Antikörpern, umfaßt;
- geeignete Mengen oder Dosen einer geeigneten Nachweissubstanz.
